# EUROPÄISCHE PATENTANMELDUNG

(11) **EP 3 375 406 A1**
(43) Veröffentlichungstag der Anmeldung: **19.09.2018**
(21) Anmeldenummer: 17161602.2
(22) Anmeldetag: 17.03.2017
(51) Int. Cl.: A61C 9/00, A61B 5/107, A61B 5/00

(54) **VORRICHTUNG UND VERFAHREN ZUM OPTISCHEN ERFASSEN VON DREIDIMENSIONALEN OBERFLÄCHENGEOMETRIEN**

(71) Anmelder: a.tron3d GmbH, 9020 Klagenfurt am Wörthersee (AT)
(72) Erfinder: JESENKO, Juergen, 9587 Riegersdorf (AT)
(74) Vertreter: Beer & Partner Patentanwälte KG

(57) **Zusammenfassung**

Bei einer Vorrichtung und einem Verfahren zum optischen Erfassen von dreidimensionalen Oberflächengeometrien mit einem Handstück (1), wobei das Handstück (1) ein oder mehr Mittel (2) zum Ausgeben von Statusmeldungen aufweist, ist wenigstens ein Mittel (2) zum Ausgeben von Statusmeldungen ein Mittel (2) zum Erzeugen von Schwingungen, welches haptisch wahrnehmbare Statusmeldungen ausgibt.

## Beschreibung

Die Erfindung betrifft eine Vorrichtung und ein Verfahren zum optischen Erfassen von dreidimensionalen Oberflächengeometrien mit einem Handstück, wobei das Handstück ein oder mehr Mittel zum Ausgeben von Statusmeldungen aufweist.

In der Zahntechnik ist es für viele Aufgaben erforderlich, ein Modell der Zähne und auch anderer intraoraler Strukturen zur Verfügung zu haben. Wurden solche Modelle früher noch physisch durch Zahnabdrücke erzeugt und beispielweise als Gipsmodelle aufbewahrt, ist es heute mehr und mehr üblich, derartige Modelle virtuell zu erfassen und zu speichern. Damit kann nicht nur der Raum für das Aufbewahren der Modelle vermindert werden, sondern es wird dem Patienten auch das unangenehme Abnehmen des Zahnbadruckes erspart. Intraoralscanner, welche optisch arbeiten, um virtuelle Modelle von intraoralen Strukturen zu erzeugen, haben sich inzwischen etabliert und werden stetig verbessert. Bei diesen Verbesserungen liegt der Fokus neben der kontinuierlichen Steigerung der Präzision des erfassten Modells und der Senkung der erforderlichen Scandauer auch auf einer Erhöhung des Nutzerkomforts - nicht nur für den Patienten, sondern auch für die Person, welche den Scanner bedient, respektive ein Handstück des Scanners führt.

Ein wesentliches Element bei der Verbesserung der Nutzerfreundlichkeit ist die möglichst einfache Kommunikation von Feedback vom Scanner zur Bedienungsperson.

Der Erfindung liegt daher die Aufgabe zu Grunde, eine verbesserte Möglichkeit zur Verfügung zu stellen, um dem Nutzer eines Intraoralscanners Feedback zu geben.

Gelöst wird diese Aufgabe erfindungsgemäß durch eine Vorrichtung mit den Merkmalen des Anspruchs 1 sowie ein Verfahren mit den Merkmalen des Anspruchs 3.

Wenn virtuelle Modelle von intraoralen Strukturen durch das optische Erfassen ihrer Oberflächengeometrien erzeugt werden, können neben den eigentlichen Daten über die Geometrie der Modelle verschiedene zusätzliche und hilfreiche Daten, beispielsweise zum Status, zur Genauigkeit und dergleichen, erzeugt und wiedergegeben werden. Im Stand der Technik erfolgt die Wiedergabe des Status und gegebenenfalls zusätzlicher Informationen und/oder Handlungsanweisungen üblicherweise visuell, in selteneren Fällen auch akustisch. Beide Methoden bringen aber Nachteile für den Nutzer mit sich. Akustische Signale werden häufig als störend empfunden. Optische Signale können die Aufmerksamkeit des Anwenders ablenken.

Erfindungsgemäß ist daher vorgesehen, dass wenigstens ein Mittel zum Geben von Statusmeldungen ein Mittel zum Erzeugen von Schwingungen, insbesondere von Vibrationen, ist. So können dem Nutzer neben den bekannten visuellen und akustischen Signalen zusätzlich oder auch ausschließlich haptische Signale gegeben werden.

Signale und Statusmeldungen schließen dabei im Sinne der Erfindung allgemeines Feedback und/oder auch Handlungsanweisungen ein.

In einer besonders bevorzugten Ausführungsform der Erfindung werden die haptischen Signale ausgegeben, wenn die Qualität der Aufnahme einen vorgegebenen Schwellwert unterschreitet. Diese Maßnahme mag selbst für den Fachmann paradox scheinen, da intuitiv angenommen wird, dass die Qualität optisch erfasster Daten nur noch weiter verschlechtert wird, wenn das Handstück in Schwingung versetzt wird. Es hat sich allerdings gezeigt, dass ab einer hinreichend hohen Bildrate der Scannersensoren genau der gegenteilige Effekt erzielt werden kann. Statt die Aufnahmen zu verschlechtern führt das Oszillieren des Handstücks und damit auch der darin befindlichen Sensoren zu einer Verbesserung der Aufnahme.

In Schwingung versetzte Sensoren erfassen die Oberfläche aus mehreren, leicht verschiedenen Positionen und schaffen damit eine höhere Dichte an geometrischen Informationen über die erfasste Oberfläche als Sensoren, die sich im Vergleich zu schwingenden Sensoren in Ruhe befinden. Unter "in Ruhe" im Vergleich zu "schwingend" wird dabei auch die langsame Bewegung des Handstücks verstanden, welches durch eine Bedienungsperson geführt wird.

Wenn das Handstück also bei einer Verschlechterung der Aufnahmequalität in Schwingung versetzt wird, wird nicht nur der Bedienungsperson die haptische Information über die Verschlechterung ausgegeben, sondern gleichzeitig bereits eine Maßnahme gesetzt, um die Verschlechterung zu beheben.

Selbstverständlich ist es möglich, mehr als nur die Signale "Handstück vibriert" und "Handstück vibriert nicht" zu geben.

Eine erste Möglichkeit die Signale zu variieren, ist die Intensität der Schwingungen zu verändern oder definierte Abfolgen von Schwingen und nicht Schwingen zu erzeugen. Diese Möglichkeit ist vor allem deshalb vorteilhaft, weil sie einfach zu implementieren ist. Allerdings wird dabei nicht das volle Potential von haptischen Ausgaben genutzt.

Während akustische und visuelle Signale nur mit sehr großem technischen Aufwand so wiedergegeben werden können, dass sie für eine Bedienungsperson dreidimensional wahrgenommen werden können, wie gemäß einer bevorzugten Weiterbildung der Erfindung vorgesehen, können verschiedene im Handstück räumlich versetzt angeordnete Mittel zum Erzeugen von Schwingungen auf einfache Art und Weise Informationen dreidimensional ausgeben.

Durch die räumlich versetzte Anordnung verschiedener Mittel zum Erzeugen von Schwingungen im Handstück können auch beispielsweise Richtungsangaben ausgegeben werden, und der Nutzer kann beispielsweise gezielt zu Bereichen des Objektes gelenkt werden, die eine bestimmte Datenqualität noch nicht erfüllen. Die hierfür notwendige Information, wo sich das Handstück gerade relativ zum zu erfassenden Objekt befindet, steht dabei automatisch zur Verfügung, da diese bei optischen Verfahren ohnehin ermittelt wird. Handlungsanweisungen, wie "Handstück weiter vor bewegen" oder "Handstück nach links neigen", können dabei von der Bedienungsperson, welche das Handstück hält, intuitiv ertastet und verstanden werden, indem entsprechende Bereiche des Handstücks in Schwingung versetzt werden.

Gemäß einer bevorzugten Durchführungsform der Erfindung erfolgt das Erfassen in verschiedenen Verfahrens-Zuständen und bei einer Änderung des Zustandes ändert sich das ausgegebene haptische Signal. Die verschiedenen Zustände entsprechen dabei verschiedenen Betriebsmodi, wie beispielsweise "normale Aufnahme" oder "Aufnahme nach einem Abriss" (Recovery), das heißt nach gegebenenfalls unbeabsichtiger Unterbrechung der Aufnahme. So kann die Bedienungsperson beispielsweise nicht nur darüber informiert werden, dass nun mit einem anderen Betriebsmodus gescannt wird, sondern auch darüber, dass es überhaupt zu einem Abriss des Scans gekommen ist.

Ein Ändern des haptischen Signals im Sinne der Erfindung kann dabei auch das Einsetzen bzw. Beginnen oder Enden des Signals bedeuten.

In einer weiteren bevorzugten Durchführungsform der Erfindung erfolgt das Erfassen in wenigstens zwei definierten physischen Bereichen, in welchen sich das Handstück befinden kann, und das ausgegebene haptische Signal ändert sich bei einem Wechsel des Bereiches, in welchem sich das Handstück befindet. Dabei kann die Bedienungsperson beispielsweise darüber informiert werden, ob sich das Handstück zu nah oder zu weit entfernt zum zu erfassenden Objekt befindet. In einer beispielhaften, sehr einfachen Umsetzung dieser Ausführungsform wird lediglich ein Bereich definiert, in welchem optimale Aufnahmen gemacht werden können und das Verlassen des Bereiches wird als Wechsel in den Bereich interpretiert, in dem keine optimalen Aufnahmen mehr möglich sind. In einer Weiterbildung der Ausführungsform können aber beispielsweise Bereiche definiert werden, denen neben "nicht optimal" auch die Eigenschaften "zu nah" oder "zu weit weg" zugewiesen werden. Unterschiedliche haptische Signale für zu große oder zu geringe Entfernung vom Objekt können dann entsprechend an die Bedienungsperson gegeben werden.

In einer weiteren bevorzugten Durchführungsform des Verfahrens führt das Handstück während des Erfassens eine Bewegung aus, werden der Bewegung bestimmte Eigenschaften zugewiesen und ändert sich bei einer Änderung der Eigenschaft der Bewegung das ausgegebene haptische Signal. So kann die Bedienungsperson beispielsweise darüber informiert werden, dass das Handstück zu schnell oder zu langsam bewegt wird.

Weitere bevorzugte Ausführungsformen der Erfindung sind Gegenstand der übrigen Unteransprüche.

Nachstehend ist ein bevorzugtes und den Schutzbereich der Ansprüche nicht beschränkendes Ausführungsbeispiel der Erfindung anhand der Zeichnung näher beschrieben. Es zeigt:

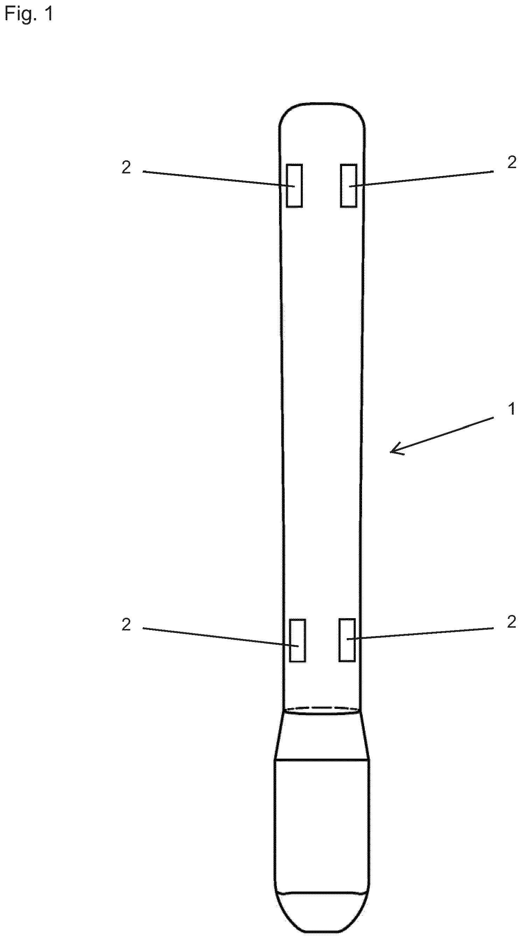

- Fig. 1: ein schematisch dargestelltes Handstück mit symbolisch eingezeichneten Mitteln zum Erzeugen von Schwingungen.

In einem schematisch dargestellten Handstück 1 eines Intraoralscanners sind symbolisch vier Mittel 2 zum Erzeugen von Schwingungen eingezeichnet. Für die Erfindung spielt es keine Rolle, welche Mittel zum Erzeugen von Schwingungen gewählt werden. Es können beispielsweise Unwuchtmotoren oder auch elektroreaktive Polymere eingesetzt werden.

Die Anzahl der Mittel 2 und der Ort, an dem die Mittel 2 angebracht sind, ist ebenso variabel. Eine Kombination mit optischen und/oder akustischen Mitteln zum Ausgeben von Informationen, Feedback, Handlungsanweisungen und dergleichen ist ebenso möglich.

## Patentansprüche

1. Vorrichtung zum optischen Erfassen von dreidimensionalen Oberflächengeometrien mit einem Handstück (1), wobei das Handstück (1) ein oder mehr Mittel (2) zum Ausgeben von Statusmeldungen aufweist, **dadurch gekennzeichnet, dass** wenigstens ein Mittel (2) zum Ausgeben von Statusmeldungen ein Mittel (2) zum Erzeugen von Schwingungen ist.

2. Vorrichtung nach Anspruch 1, **dadurch gekennzeichnet, dass** im Handstück (1) zwei oder mehr Mittel (2) zum Erzeugen von Schwingungen räumlich versetzt, insbesondere diagonal zueinander und/oder einander gegenüberliegend angeordnet sind.

3. Verfahren zum optischen Erfassen von dreidimensionalen Oberflächengeometrien mit einem Handstück, wobei das Handstück (1) ein oder mehr Mittel (2) zum Ausgeben von Statusmeldungen aufweist, **dadurch gekennzeichnet, dass** wenigstens ein Mittel (2) Statusmeldungen als haptische Signale durch Schwingungen ausgibt.

4. Verfahren nach Anspruch 3, **dadurch gekennzeichnet, dass** die haptischen Signale ausgegeben werden, wenn die Qualität der Aufnahme einen vorgegebenen Schwellwert über- oder unterschreitet.

5. Verfahren nach Anspruch 3 oder 4, **dadurch gekennzeichnet, dass** verschiedene, räumlich versetzte Mittel (2) zum Ausgeben haptischer Signale verschieden intensiv und/oder zeitlich versetzt schwingen, um Richtungsinformationen auszugeben.

6. Verfahren nach einem der Ansprüche 3 bis 5, **dadurch gekennzeichnet, dass** das Erfassen in verschiedenen Verfahrens-Zuständen erfolgt und dass sich bei einer Änderung des Verfahrens-Zustandes das ausgegebene haptische Signal ändert.

7. Verfahren nach einem der Ansprüche 3 bis 6, **dadurch gekennzeichnet, dass** das Erfassen in wenigstens zwei definierten physischen Bereichen, in welchen sich das Handstück (1) befinden kann, erfolgt und dass sich das ausgegebene haptische Signal bei einem Wechsel des Bereiches, in welchem sich das Handstück befindet, ändert.

8. Verfahren nach einem der Ansprüche 3 bis 7, **dadurch gekennzeichnet, dass** das Handstück während des Erfassens eine Bewegung ausführt, dass der Bewegung bestimmte Eigenschaften zugewiesen werden und dass sich das ausgegebene haptische Signal bei einer Änderung der Eigenschaft der Bewegung ändert.

9. Verfahren nach einem der Ansprüche 3 bis 8, **dadurch gekennzeichnet, dass** das Mittel (2) haptische Signale in verschiedenen Intensitäten, Frequenzen und Abfolgen ausgibt.

10. Verfahren nach einem der Ansprüche 3 bis 9, **dadurch gekennzeichnet, dass** das Erfassen mit einer Bildfrequenz eines Sensors erfolgt, und dass die Frequenz des haptischen Signals an die Bildfrequenz des Sensors angepasst wird.
